# EUROPÄISCHE PATENTSCHRIFT

(11) **EP 3 643 240 B1**
(45) Veröffentlichungstag und Bekanntmachung des Hinweises auf die Patenterteilung: **17.03.2021**
(21) Anmeldenummer: 18202333.3
(22) Anmeldetag: 24.10.2018
(51) Int. Cl.: A61B 6/04, A61B 6/00, A61B 5/055, G06F 3/01, G06F 3/041

(54) **MEDIZINISCHE BILDGEBUNGSEINRICHTUNG UND VERFAHREN ZUM BETRIEB EINER MEDIZINISCHEN BILDGEBUNGSEINRICHTUNG**
MEDICAL IMAGING DEVICE, AND METHOD FOR OPERATING A MEDICAL IMAGING DEVICE
APPAREIL D'IMAGERIE MÉDICALE ET PROCÉDÉ DE FONCTIONNEMENT D'UN APPAREIL D'IMAGERIE MÉDICALE

(43) Veröffentlichungstag der Anmeldung: 29.04.2020
(73) Patentinhaber: Siemens Healthcare GmbH, 91052 Erlangen (DE)
(72) Erfinder: Höcht, Philipp, 91207 Lauf (DE); Wolf, Felix, 91088 Bubenreuth (DE)

(56) Entgegenhaltungen:
- EP-A1- 3 348 201
- DE-A1-102015 204 767
- US-A1- 2015 277 420

## Beschreibung

Die Erfindung betrifft eine medizinische Bildgebungseinrichtung, insbesondere eine Computertomographieeinrichtung und/oder eine Magnetresonanzeinrichtung, aufweisend wenigstens eine mittels eines Aktor bewegbare und/oder mittels eines Einstellmittels einstellbare Komponente, eine Bedieneinrichtung zur Steuerung des Betriebs der wenigstens einen Komponente und eine der Bedieneinrichtung zugeordnete Steuereinrichtung. Daneben betrifft die Erfindung ein Verfahren zum Betrieb einer solchen medizinischen Bildgebungseinrichtung.

Medizinische Bildgebungseinrichtungen, insbesondere größere Anlagen wie Magnetresonanzeinrichtungen und/oder Röntgeneinrichtungen, weisen üblicherweise Komponenten auf, die über Aktoren und/oder Einstellmittel bewegbar beziehungsweise einstellbar sind. Ein Beispiel für eine derartige Komponente ist eine Patientenliege, die beispielsweise in eine Gantry und/oder eine Patientenaufnahme eingefahren werden kann. Gerade bei Röntgeneinrichtungen können auch weitere bewegbare Komponenten existieren, beispielsweise Anteile der Aufnahmeanordnung und/oder Trägerelemente, beispielsweise ein C-Bogen bei einer Röntgeneinrichtung mit einem C-Bogen als medizinische Bildgebungseinrichtung. Einstellbare Komponenten umfassen beispielsweise Beleuchtungskomponenten, die in ihrer Helligkeit einstellbar sein können, Klimatisierungseinrichtungen, beispielsweise Lüfter, die in ihrer Intensität einstellbar sein können, und dergleichen. Selbstverständlich können auch Komponenten vorliegen, die bewegbar und einstellbar sind.

Hierbei ist es eine Besonderheit medizinischer Bildgebungseinrichtungen, dass der Blick eines Bedieners beim Durchführen einer Bedienhandlung meist nicht auf die Bedieneinrichtung beziehungsweise konkret ein entsprechendes Bedienmittel gerichtet ist, sondern auf die zu bedienende Komponente. Beispielsweise wird bei einer Patientenliege optisch darauf geachtet, dass die Sicherheit des darauf gelagerten Patienten gewährleistet ist und die Zielposition korrekt erreicht wird. Dies hat diverse Anforderungen an Bedieneinrichtungen von medizinischen Bildgebungseinrichtungen zur Folge.

Nichtsdestotrotz wurde für medizinische Bildgebungseinrichtungen, beispielsweise Magnetresonanzeinrichtungen, inzwischen auch vorgeschlagen, einen Touchscreen (Touch-Display) als Bedienmittel neben anderen Bedienmitteln einer Bedieneinrichtung einzusetzen. Während in sonstigen Bereichen bei der Bedienung eines Touchscreens die volle Aufmerksamkeit dem Touchscreen gewidmet wird, beispielsweise bei Smartphones, Tablets und Bankautomaten, liegt bei der Bedienung einer medizinischen Bildgebungseinrichtung die Aufmerksamkeit auf dem Patienten beziehungsweise den Komponenten, so dass Funktionalitäten, wie beispielsweise das Verfahren der Patientenliege, idealerweise "blind" möglich sein sollen, das heißt unter zumindest hauptsächlichem Verweilen des Blickes auf den Patienten und/oder der Komponente. Dies ist mit über Touchscreens realisierten Schaltflächen, Reglern und/oder sonstigen Bedienelementen nicht ohne weiteres möglich, so dass weitere Bedienmittel/Bedieneinheiten eingesetzt werden.

Mithin wurden beispielsweise bei herkömmlichen Magnetresonanzeinrichtungen zur Bedienung bewegbarer und/oder einstellbarer Komponenten Bedienmittel in einer festen geometrischen Anordnung und mit bei der Betätigung eines Bedienelements gegebenem haptischem Feedback vorgeschlagen. Eine konkrete, bekannte Ausgestaltung sieht beispielsweise vor, Bedienmittel mit mehreren, teils zweistufigen, Kurzhubtasten und/oder Drehrädern mit Rückstellfeder ("Jogwheel") zu verwenden. Dabei existiert haptisches Feedback beispielsweise beim Druck einer Kurzhubtaste sowie bei einem Auslenken des Drehrades, so dass sich die medizinische Bildgebungseinrichtung mit etwas Übung ohne hinzuschauen oder nur mit kurzem Blick auf die Bedienmittel bedienen lässt.

Bezüglich der Verwendung von Touchscreens wurde vorgeschlagen, eine Strukturierung in die Glasoberfläche des Touchscreens einzubringen, beispielsweise Mulden und/oder Erhebungen vorzusehen, die zur Realisierung bestimmter Funktionen, beispielsweise des Verschiebens der Patientenliege, "ertastet" werden können beziehungsweise aufgrund der Führung in einer Mulde "blind" bedient werden können. Dabei erfolgt jedoch nachteilhafter Weise die Festlegung auf eine bestimmte Funktion an einem bestimmten Ort des Touchscreens. Ferner lassen sich bestimmte Funktionalitäten schlecht unterscheiden und der Aufbau und die Fertigung werden komplexer.

Eine andere bei der Auslegung von Bedieneinrichtungen für medizinische Bildgebungseinrichtungen zu beachtende Besonderheit ist die hinsichtlich des Patienten notwendige hohe Sicherheit. Dies gilt insbesondere für Patientenliegen und/oder sonstige bewegte Komponenten. Für bewegte Komponenten, insbesondere für Patientenliegen, wurden dabei unterschiedliche Realisierungen von Verfahrmöglichkeiten vorgeschlagen, beispielsweise Zielfahrten (ZF), bei denen als Bedienhandlung ein Kommando für ein bestimmtes Ziel angegeben wird, und kontinuierliche Fahrten (KF), bei denen solange bewegt wird, bis ein der Bewegung zugeordnetes Bedienelement wieder losgelassen wird. Dabei werden meistens, wie bereits dargelegt, gezielt mechanische Bedienelemente, beispielsweise Kurzhubtasten und Drehräder mit Rückstellfeder, eingesetzt.

Zur Sicherstellung einer fehlerfreien Bedienung, gerade bei den Patientenliegen, wurden bereits einige Maßnahmen für Bedieneinrichtungen vorgeschlagen. So können beispielsweise mechanisch vorgesehene Stopp-Schalter zum Beenden der Bewegung für bewegbare Komponenten, insbesondere Patientenliegen, erstfehlersicher ausgelegt werden, insbesondere also durch redundante Betätigungsdetektion. Erstfehlersicherheit wird dabei insbesondere so verstanden, dass kein erster Fehler ein inakzeptables Risiko für den Patienten verursachen darf.

Für Bedienelemente, denen eine Zielfahrt zugeordnet ist, kann beispielsweise vorgesehen sein, das entsprechende Bedienelement zunächst für eine bestimmte Zeitspanne zu drücken, bevor der entsprechende Zielfahrbefehl ausgelöst wird. Hierdurch wird ein versehentliches Auslösen vermieden. Für kontinuierliche Fahrten von Komponenten wurde vorgeschlagen, eine Art "Totmannschaltung" zu realisieren, das bedeutet, es wird kontinuierlich ein Verfahrkommando gesendet, solange das zugeordnete Bedienelement betätigt wird. Für die Firmware der Bedieneinrichtungen sind ebenfalls bestimmte Software-Sicherheitsklassen vorgesehen, die umgesetzt werden können.

Konkret wurde auch bereits ein erstfehlersicherer Touchscreen für medizinische Bildgebungseinrichtungen vorgeschlagen, der zwei Berührungssensoren verwendet, nämlich einen resistiven sowie einen kapazitiven Berührungssensor. Beispielhaft sei diesbezüglich auf DE 10 2012 207 114 A1 verwiesen.

Aus EP 3 348 201 A1 ist ein Verfahren zu einem Positionieren einer Patientenliege bekannt. Dabei wird zumindest ein Bewegungsfreiheitsgrad der Patientenliege in Abhängigkeit einer vom Benutzer auf das Bedienelement ausgeübten Kraft verstellt.

Aus DE 10 2015 204 767 A1 ist eine Bedieneinheit für ein Medizingerät bekannt mit einer Bedieneinheit. Hierbei wird mittels Drucksensoren eine Bedienfunktion eines Benutzers erkannt. Zudem ist auch aus US 2015 277 420 A1 ein Medizingerät bekannt, bei dem mittels berührempfindlichen Sensoren ein Bediensignal erfasst wird.

Der Erfindung liegt die Aufgabe zugrunde, eine bezüglich der Sicherheit und/oder Bedienbarkeit einer medizinischen Bildgebungseinrichtung verbesserte Möglichkeit zur Realisierung eines berührungssensitiven Bedienmittels anzugeben.

Diese Aufgabe wird gelöst durch eine medizinische Bildgebungseinrichtung und ein Verfahren zum Betrieb einer medizinischen Bildgebungseinrichtung gemäß den unabhängigen Ansprüchen. Vorteilhafte Ausgestaltungen ergeben sich aus den Unteransprüchen.

Bei einer medizinischen Bildgebungseinrichtung der eingangs genannten Art ist erfindungsgemäß vorgesehen, dass die Bedieneinrichtung ein wenigstens einen Berührungssensor und wenigstens einen die Stärke der Berührung vermessenden Kraftsensor aufweisendes, berührungs- und kraftsensitives, flächiges Bedienmittel aufweist, wobei die die Steuereinrichtung ausgebildet ist, bei Detektion wenigstens einer ersten Bedienhandlung abhängig von der Berührposition der ersten Bedienhandlung wenigstens zwei Bedienbereiche der Bedienfläche des Bedienmittels zu definieren, in denen jeweils unterschiedlichen Funktionalitäten zugeordnete zweite Bedienhandlungen detektierbar sind.

Mit anderen Worten wird also vorgeschlagen, ein sogenanntes Force-Touch-Bedienmittel als Teil der Bedieneinrichtung einzusetzen, mittels welchem sowohl Berührungen der Bedienfläche des Bedienmittels festgestellt werden können als auch die Stärke dieser Berührungen als Berührkraft, wozu zusätzlich zu einem Berührungssensor auch ein Kraftsensor eingesetzt wird. Dabei ist der Begriff "Kraftsensor" breit zu verstehen als ein Sensor, der eine Kraft beschreibende Messdaten aufnimmt. Beispiele umfassen neben "klassischen" Kraftaufnehmern auch Beschleunigungssensoren, Auslenkungssensoren, die beispielsweise die Auslenkung eines Federelementes erfassen, und dergleichen.

Die Steuereinrichtung ist nun ausgebildet, Sensordaten sowohl des Berührungssensors als auch des Kraftsensors auszuwerten, um Bedienhandlungen feststellen zu können und eine entsprechende Ansteuerung der Aktoren und/oder Einstellmittel sowie gegebenenfalls anderer Bauteile der medizinischen Bildgebungseinrichtung zu bewirken. Dabei weist das Bedienmittel bevorzugt als Bedienfläche eine Platte auf, insbesondere eine Glasplatte. Der Platte kann der beispielsweise als Piezosensor und/oder Dehnungsmessstreifen ausgebildete Kraftsensor zugeordnet sein, wobei meist mehrere Kraftsensoren verwendet werden, um eine Berührpositionsmessung auch mittels der Kraftsensoren durchführen zu können. Der Berührsensor kann beispielsweise als kapazitiver Berührsensor ausgelegt sein. Eine Berührkraftmessung kann auch unter Berücksichtigung einer Kapazitätsänderung erfolgen.

Eine konkrete Ausgestaltung mit einer im Wesentlichen rechteckigen Platte kann beispielsweise vorsehen, dass in jeder Ecke der Platte jeweils ein Kraftsensor, insbesondere ein Piezoelement, vorgesehen ist, so dass aufgrund der unterschiedlichen Krafteinträge in die verschiedenen Kraftaufnehmer (also Kraftsensoren) nicht nur ein die insgesamte Stärke der Berührung beschreibendes Auswertungsergebnis erhalten werden kann, sondern aufgrund der Verteilung der Krafteinträge auch eine Position des Berührereignisses als "Kraft-Event" geometrisch ermittelt werden kann.

Das Vorsehen von Force-Touch-Bedienmitteln als Teil der Bedieneinrichtung ermöglicht es zum einen, die Sicherheit bei der Einstellung und/oder Bewegung von Komponenten zu erhöhen, zum zweiten über entsprechend umgesetzte Bedienhandlungen auch eine Bedienung von Komponenten ohne Blick auf das Bedienmittel zu erlauben, und schließlich die Flexibilität bezüglich möglicher Bedienhandlungen zu erhöhen, da mit der von dem Kraftsensor vermessenen Stärke der Berührung ein weiterer, Bedienhandlungen charakterisierender Parameter vorliegt. Konkrete Umsetzungen dieser beschriebenen Vorteile werden im Folgenden noch genauer erläutert. Nachdem das Bedienmittel insbesondere als Touchscreen (mit als Display eingebauter Anzeigeeinrichtung) realisiert werden kann und zudem bei "blinder" Bedienbarkeit über das Bedienmittel keine weiteren, beispielsweise haptisch ertastbaren und/oder mechanisch haptisch rückmeldenden Bedienelemente/Bedienmittel an der Bedieneinrichtung erforderlich sind, kann eine komplette Bedienung der medizinischen Bildgebungseinrichtung über das insbesondere als Touchscreen ausgebildete Bedienmittel erfolgen. Der Kontakt zum Patienten wird verbessert, indem das Bedienmittel ohne Hinschauen bedient werden kann. Das Wegfallen weiterer Bedienmittel beziehungsweise Bedienelemente kann ferner zu einer Kostenersparnis führen, da durch die Ausgestaltung eines Touch-Bedienmittels als Force-Touch-Bedienmittel nur ein geringer Aufpreis erforderlich ist.

Dabei bezieht sich die Erfindung bevorzugt auf bewegte Komponenten der medizinischen Bildgebungseinrichtung, insbesondere die Patientenliege und/oder sonstige, im Bereich des Patienten bewegbare Komponenten. Sie ist jedoch auch vorteilhaft anwendbar auf sonstige Komponenten, die der Bewegung und/oder Einstellung mittels der Bedieneinrichtung zugänglich sind, beispielsweise Beleuchtungskomponenten, Klimatisierungskomponenten wie Lüfter, und dergleichen.

In besonders bevorzugter Ausgestaltung hinsichtlich der Erhöhung der Bediensicherheit kann erfindungsgemäß vorgesehen sein, dass dem Kraftsensor und dem Berührungssensor jeweils eigene Controller zugeordnet sind und/oder die Steuereinrichtung zum Detektieren wenigstens einer möglichen, insbesondere auf die Bewegung einer Komponente bezogenen, Bedienhandlung nur bei, insbesondere innerhalb eines Toleranzbereichs übereinstimmenden, Berührpositionsdaten des Berührungssensors und des wenigstens einen auch zur Berührpositionsmessung ausgebildeten Kraftsensors und/oder nur bei Überschreiten einer Mindestberührkraft und/oder einer Mindestberührzeitdauer ausgebildet ist. Dabei ist es, wie bereits dargelegt, besonders bevorzugt, wenn zur kraftsensorseitigen Berührpositionsmessung mehrere Kraftsensoren an unterschiedlichen Verbaupositionen des flächigen Bedienmittels vorgesehen sind, deren jeweilig gemessene Teilkräfte in dem zugeordneten Controller und/oder der Steuereinrichtung zur Ermittlung einer Berührposition und einer Berührkraft ausgewertet werden.

Insbesondere im Hinblick auf eine redundante Berührpositionsmessung ist es mithin möglich, ähnlich wie bei bekannten, kapazitive und resistive Berührungssensoren nutzenden Touchscreens eine Erstfehlersicherheit zumindest bezüglich ausgewählter, der Sicherheit des Patienten zugeordneter Bedienhandlungen herzustellen, indem die redundante Ermittlung ausgenutzt wird und ein Vergleich durchgeführt, wobei nur dann, wenn die Berührpositionen, insbesondere innerhalb eines Toleranzbereichs, übereinstimmen, tatsächlich eine entsprechende Bedienhandlung angenommen werden kann. Auf diese Weise kann beispielsweise Fehlauslösungen durch Messfehler oder dergleichen entgegengewirkt werden. Dies wird durch die unabhängige Auswertung der Rohdaten des Berührungssensors und des Kraftsensors in unabhängigen Controllern noch unterstützt, wobei auch alleinstehend eine unabhängige Auswertung zweckmäßig ist. Mit besonderem Vorteil zusätzlich zu einer Ausnutzung der Redundanz bei einer Berührpositionsmessung können zudem als weitere Bedingungen für zumindest einen Teil der möglichen Bedienhandlungen, insbesondere der Bewegung von Komponenten zugeordneten Bedienhandlungen, bestimmte, insbesondere weitere, Anforderungen gestellt werden. Beispielsweise kann das Überschreiten einer gewissen Minimalkraft gefordert werden, um eine Bedienhandlung erkennen zu können, so dass leichte, versehentliche Berührungen keine Bedienhandlung darstellen und somit keine Steuermaßnahme auslösen.

Im Beispiel einer Patientenliege als Komponente werden Fahrten der Patientenliege mithin erst dann ausgelöst, wenn alle Bedingungen eingehalten sind. Beispielsweise können bei den eingangs erläuterten CTM-Fahrten kontinuierlich Verfahrkommandos gesendet werden, solange die Bedingungen für die zugeordnete Bedienhandlung eingehalten werden. Bei CTM-Fahrten können den entsprechenden Bedienhandlungen auch zusätzliche Bedingungen, beispielsweise im Vergleich zu CTM-Fahrten, zugeordnet werden, beispielsweise das Überschreiten einer gewissen Zeitdauer, für die die Bedienungen eingehalten werden müssen. Eine derartige Mindestberührzeitdauer kann beispielsweise 0,8 bis 2 Sekunden, bevorzugt 1 Sekunde, betragen. Zusätzlich zu den beispielhaft genannten Bedingungen, die unterschiedlichen Bedienhandlungen getrennt zugeordnet sein können, sind selbstverständlich auch weitere Bedingungen denkbar, die sich auch in den Bedienhandlungen selbst ausdrücken können, welche aber die Bediensicherheit weiter erhöhen. Beispielsweise können Bedienhandlungen für kritische Bewegungen und/oder Einstellungen gezielt als Multi-Touch/Force-Bedienhandlungen und/oder bestimmte Gesten, also Bewegungen auf der Bedienfläche, umfassend angefordert werden. Multi-Touch/Force-Events als Bedienhandlung anzufordern, kann auch als eine Art "Safe Touch" verstanden werden, das bedeutet, ein bislang beispielsweise als eigenes, zusätzliches Bedienmittel realisiertes Bedienelement, welches zusätzlich zu einer Bedienung über beispielsweise ein Touchscreen erforderlich war, kann wegfallen, wenn die redundante Auslegung des Bedienmittels, speziell des Touchscreens, sichergestellt werden kann.

Wie bereits erwähnt, erlaubt die zusätzliche Feststellung der Stärke der Berührung durch den Kraftsensor weitere, zweckmäßige Funktionalitäten. So kann beispielsweise vorgesehen sein, dass die Steuereinrichtung zur insbesondere stufenlosen Anpassung wenigstens eines einer Bedienhandlung zugeordneten Steuerparameters, insbesondere einer Bewegungsgeschwindigkeit, in Abhängigkeit von der zu der Bedienhandlung gemessenen Berührkraft ausgebildet ist. Die Verwendung eines Force-Touch-Bedienmittels erlaubt also die stufenlose Variation einer Größe, beispielsweise der Bewegungsgeschwindigkeit einer Patientenliege, abhängig von der Kraft, mit der das Bedienmittel gedrückt wird.

Besonders vorteilhaft ist es im Rahmen der vorliegenden Erfindung auch, wenn die Steuereinrichtung zur Unterscheidung von Bedienhandlungen aufgrund einer Berührkraft und/oder Berührdauer und/oder zur Erkennung eines innerhalb eines Zeitraums erfolgenden, zweifachen Berührens als Bedienhandlung, insbesondere zum Wechsel eines Bedienmodus, ausgebildet ist. Insbesondere für diese Ausgestaltungen gilt, dass zur Identifikation einer Berührtätigkeit als eine Bedienhandlung, der eine Steuermaßnahme zugeordnet ist, zusätzlich selbstverständlich auch die Berührposition berücksichtigt werden kann, beispielsweise zum Zuordnen zu einem Bedienbereich der Bedienfläche des Bedienmittels.

Beispielsweise können also Bedienhandlungen auch abhängig von der bei der Berührung aufgebrachten Kraft unterschieden werden, was erst durch das Vorsehen eines Kraftsensors zweckmäßig ermöglicht wird und somit eine bessere Differenzierung von Bedienhandlungen/eine größere Zahl unterschiedlicher Bedienhandlungen ermöglicht, erst recht in Zusammenhand mit der Betrachtung einer Bediendauer. Besonders zweckmäßig kann die Erkennung eines innerhalb eines Zeitraums erfolgenden, zweifachen Berührens als Bedienhandlung sein, da auf diese Weise eine Art "Doppelklick" über das Bedienmittel realisiert sein kann, was insbesondere zur Anwahl bestimmter Betriebsmodi ohne Wenden des Blicks auf das Bedienmittel zweckmäßig eingesetzt werden kann. Beispielsweise kann ein solcher "Doppelklick" zur Aktivierung eines Betriebsmodus zum Verfahren einer bestimmten Komponente, insbesondere einer Patientenliege, führen.
In einer besonders vorteilhaften Ausgestaltung der vorliegenden Erfindung ist vorgesehen, dass die Steuereinrichtung zur Auswertung eines Berührkraftverlaufs über die Zeit zur Detektion einer Bedienhandlung ausgebildet ist. Das bedeutet, es wird ein Zeit-Kraft-Verlauf analysiert, um bestimmte Bedienhandlungen feststellen zu können und insbesondere von anderen Bedienhandlungen, die andere Berührkraftverläufe über die Zeit aufweisen, unterscheiden zu können. Eine besonders bevorzugte konkrete Ausgestaltung in diesem Kontext sieht vor, dass die Steuereinrichtung zur Detektion einer Notfall-Bedienhandlung, insbesondere eines Not-Bewegungsstopps, bei einem impulsartigen Berührkraftverlauf, bei dem mit einer einen Steigungsschwellwert überschreitenden Steigung und/oder in einer einen Zeitschwellwert unterschreitenden Zeit eine einen Kraftschwellwert überschreitende Berührkraft erreicht wird, ausgebildet ist. Ein derartiger, impulsartiger Berührkraftverlauf entsteht beispielsweise, wenn der Bediener, insbesondere auch auf einer größeren Fläche, auf die Bedienfläche des Bedienmittels schlägt. Ein derartiges "Draufhauen" kann seitens der Steuereinrichtung erkannt und einer Notfall-Steuermaßnahme, beispielsweise einem Notfall-Bewegungsstopp, zugeordnet sein. Auf diese Weise kann ein Notfall-Bedienelement auch durch das Bedienmittel bereitgestellt werden.

Erfindungsgemäß ist es vorgesehen, dass die Steuereinrichtung ausgebildet ist, bei Detektion wenigstens einer ersten Bedienhandlung abhängig von der Berührposition der ersten Bedienhandlung wenigstens zwei Bedienbereiche der Bedienfläche des Bedienmittels zu definieren, in denen jeweils unterschiedlichen Funktionalitäten zugeordnete zweite Bedienhandlungen detektierbar sind. Diese Ausgestaltung ermöglicht mit besonderem Vorteil eine räumliche Entkopplung der Bedientätigkeit von vorgegebenen Positionen auf der Bedienfläche des Bedienmittels. Denn letztlich ist eine erste, mit besonderem Vorteil ohne Hinsehen durchführbare Bedienhandlung ausreichend, um einen Betriebsmodus zu aktivieren, in dem dynamisch Bedienbereiche geschaffen werden, die bestimmten Funktionalitäten zugeordnet sind und deren Lage dem Bediener ja bekannt ist, nachdem ihm die Berührposition der ersten Bedienhandlung bekannt ist. Mithin ist ein Hinsehen auch weiterhin nicht erforderlich. Ist eine Anzeigeeinrichtung vorgesehen, beispielsweise also das Bedienmittel als ein Touchscreen ausgebildet, kann auch eine Anzeige der Anzeigeeinrichtung so angepasst werden, dass die entsprechenden Bedienbereiche beschrieben sind, um auch ein optisches Feedback seitens des Bedienmittels selbst geben zu können. Mit anderen Worten bedeutet das, dass beispielsweise ein bestimmtes graphisches Overlay und oder eine sonstige Anzeigedarstellung bei Durchführung der ersten Bedienhandlung an einer beliebigen Stelle der Bedienfläche, also der Berührposition der ersten Bedienhandlung, eingeblendet werden kann, das beziehungsweise die auf eben diese Berührposition und die hierdurch definierten Bedienbereiche abgestimmt ist.

In einer konkreten Ausgestaltung dieser besonders bevorzugten Weiterbildung kann vorgesehen sein, dass durch die Steuereinrichtung wenigstens zwei bezüglich der Berührposition der ersten Bedienhandlung gegenüberliegende Bedienbereiche der Ansteuerung unterschiedlicher Bewegungsrichtungen und/oder Einstellrichtungen einer der wenigstens einen Komponente, insbesondere der Patientenliege, zugeordnet sind, wobei insbesondere die Berührkraft einer zur Bewegung der Komponente vorgesehenen zweiten Bedienhandlung steuereinrichtungsseitig zur Ermittlung einer Bewegungsgeschwindigkeit und/oder Einstellungsgeschwindigkeit auswertbar ist. Wird also beispielsweise die erste Bedienhandlung, insbesondere Drücken an einer beliebigen Berührposition mit einer vorbestimmten Berührkraft und/oder Berührdauer, ausgeführt, kann die Bewegung einer Komponente aktiviert werden, wobei beispielsweise ein in eine Richtung ausgehend von der Berührposition vorgesehener Bedienbereich der Bewegung in eine Richtung, der andere, gegenüberliegende Bedienbereich der Bewegung in eine Gegenrichtung durch entsprechende zweite Bedienhandlungen dienen kann. Dabei ist eine derartige Ausgestaltung allerdings nicht auf die Bewegung von Komponenten beschränkt, sondern kann sich auch auf andere, anderweitig einstellbare Komponenten beziehen, beispielsweise auf die Erhöhung oder Erniedrigung der Stärke eines Leuchtens einer Beleuchtungskomponente und/oder des Lüfterstroms einer Klimatisierungskomponente.

Besonders vorteilhaft ist es in diesem Zusammenhang auch, wenn die Steuereinrichtung zur Anwahl eines Bedienbereiches durch Auswertung der Richtung eines Ziehens ausgehend von der Berührposition nach der ersten Bedienhandlung ausgebildet ist. Beispielsweise kann also eine zweite Bedienhandlung unmittelbar an die erste Bedienhandlung anschließen, indem ein beispielsweise das Bedienen durchführender Finger von der Berührposition in einen Bedienbereich weiterbewegt wird, der anhand dieser Richtung angewählt werden kann.

Gerade im Zusammenhang mit den zuvor beschriebenen Möglichkeiten zur Definition von ersten Bedienhandlungen, die bestimmte Betriebsmodi aktivieren, kann sich im Rahmen der vorliegenden Erfindung mithin ein Bedienkonzept ergeben, in dem beispielsweise ausgehend von einem in einem Ruhemodus befindlichen Bedienmittel unterschiedliche erste Bedienhandlungen zu unterschiedlichen Betriebsmodi mit unterschiedlich wirkenden und/oder vorgegebenen Bedienbereichen abhängig von der Berührposition führen können. Beispielsweise ist es denkbar, über einen "Doppelklick", mithin ein innerhalb eines Zeitraums erfolgendes, zweifaches Berühren, als erste Bedienhandlung einen Bedienmodus zur Bewegung einer vorbestimmten Komponente, beispielsweise der Patientenliege, aktiviert werden. Hierbei können beispielsweise wenigstens zwei Bedienbereiche, beispielsweise von der Berührposition "nach oben" und "nach unten" definiert werden, die eine Fahrt der Patientenliege nach oben und/oder in eine Patientenaufnahme/Gantry hinein beziehungsweise nach unten und/oder aus einer Patientenaufnahme/Gantry hinaus zugeordnet sein können. Es können selbstverständlich auch weitere Bedienbereiche definiert werden, beispielsweise ein Bedienbereich "nach links", der zum Verfahren der Patientenliege in eine Grundposition führt und/oder ein Bedienbereich "nach rechts" der eine Laserpositionierungshilfe für die Patientenliege aktiviert. Zweite Bedienhandlungen innerhalb der entsprechenden Bedienbereiche führen dann zu entsprechenden Reaktionen, also der Durchführung der jeweiligen der zweiten Bedienhandlung zugeordneten Steuermaßnahmen. Wird beispielsweise der Betriebsmodus zur Bewegung der Patientenliege angewählt, beispielsweise durch den erwähnten "Doppelklick" oder durch ein mit einer bestimmten Berührkraft und/oder für eine bestimmte Zeitdauer erfolgendes Berühren der Berührposition, kann dann der Finger in einen Bedienbereich weiterbewegt werden, wo eine Bewegung mit einer Bewegungsgeschwindigkeit entsprechend der Berührkraft ausgelöst werden kann und dergleichen. Es sei angemerkt, dass selbstverständlich die erste beziehungsweise als zweite Bedienhandlung auch weitere Arten vom Bedienhandlungen denkbar sind, beispielsweise Force-Touch-Gesten, L-förmige Gesten, Multi-Touch-Gesten und dergleichen.

Im gerade angesprochenen, genannten Beispiel können auch andere erste Bedienhandlungen ausgehend vom Ruhemodus vorgesehen sein, beispielsweise zur Aktivierung eines Patientenkomfortmodus, wobei dann entsprechend abhängig von der Berührposition definierte Bedienbereiche der Einstellung einer Klimatisierungskomponente, beispielsweise eines Patientenlüfters, und/oder einer Beleuchtungskomponente, dienen können, wobei eine dieser beispielhaft genannten Komponenten dann insbesondere jeweils zwei bezüglich der Berührposition gegenüberliegende Bedienbereiche zugeordnet sind, beispielsweise "oben/unten" der Beleuchtungskomponente, "links/rechts" der Klimatisierungskomponente.

Durch Kombination der oben beschriebenen Möglichkeiten lassen sich also besonders vorteilhafte, insbesondere ohne Blick auf das Bedienmittel durchführbare Bedienhandlungen beziehungsweise Abfolgen von Bedienhandlungen definieren. Beispielsweise ist auch eine Ausgestaltung denkbar, wobei durch ein Drücken der Bedienfläche mit einer eine vorbestimmte Minimalberührkraft übersteigenden Berührkraft als erste Bedienhandlung, ein sodann stattfindendes Weiterführen des berührenden Fingers in einen Bedienbereich und dortiges Drücken mit einer Berührkraft die Bewegung einer bewegbaren Komponente angewählt wird und dann die Komponente mit einer Bewegungsgeschwindigkeit entsprechend der Berührkraft durch Ansteuerung des Aktors mittels der Steuereinrichtung bewegt wird.

In einer zweckmäßen Weiterbildung kann vorgesehen sein, dass die Bedieneinrichtung wenigstens ein dem Bedienmittel zugeordnetes, akustisches und/oder haptisches und/oder optisches Anzeigemittel zur Anzeige und/oder Bestätigung wenigstens einer Bedienhandlung aufweist. Das bedeutet, es kann ein akustisches, haptisches und/oder optisches Feedback gegeben werden, welches insbesondere geeignet ist, die "Bedienung ohne Hinschauen" zu unterstützen. Während bei manchen Bedienhandlungen das Feedback durch die Bewegung und/oder Einstellung der entsprechenden Komponente unmittelbar erkennbar ist, beispielsweise beim Start einer Bewegung der Patientenliege, beim Einschalten einer Beleuchtungskomponente oder dergleichen, kann es bei anderen, Steuermaßnahmen für Komponenten zur Folge habenden Bedienhandlungen weniger offensichtlich sein, dass die Steuermaßnahme durchgeführt wurde beziehungsweise die Bedienhandlung erkannt wurde. Hierzu kann beispielsweise ein haptisches Feedback, beispielsweise durch eine Vibrationseinrichtung an dem Bedienmittel selber, zweckmäßig sein, wobei auch akustische Anzeigemittel zweckmäßig sind, da sie unabhängig vom Blick des Bedieners wahrgenommen werden, beispielsweise Lautsprecher oder dergleichen. Besonders vorteilhaft im Hinblick auf haptische Anzeigemittel ist die Ausgestaltung der Kraftsensoren als Piezoelemente umfassend; dann können die Kraftsensoren gleichzeitig als haptisches Anzeigemittel eingesetzt werden. Doch auch optische Anzeigemittel sind einsetzbar, wobei eine bevorzugte Weiterbildung vorsieht, dass das optische Anzeigemittel wenigstens einer Komponente zugeordnet und im Blickbereich einer das Bedienmittel nutzenden Person zu der Komponente und/oder einem Patienten, entfernt von dem Bedienmittel, angeordnet ist. Das bedeutet, das optische Anzeigemittel kann auch von dem Bedienmittel entfernt im angenommenen Blickfeld des Bedieners angeordnet werden, so dass dieses zweckmäßige Blickfeld der das Bedienmittel nutzenden Person nicht verändert werden muss und dennoch eine optische Feedbackgabe vorteilhaft ermöglicht wird.

Wie bereits erwähnt, können zweckmäßige Ausführungsformen der vorliegenden Erfindung vorsehen, dass das Bedienmittel eine die Bedienfläche wenigstens teilweise abdeckende Anzeigeeinrichtung aufweist, insbesondere das Bedienmittel als ein Touchscreen ausgebildet ist. Touchscreens, die auch Kraftsensoren aufweisen, sind, beispielsweise als "Force-Touch-Display", im Stand der Technik bereits grundsätzlich bekannt geworden, beispielsweise im Hinblick auf Tablets und/oder Smartphones. Dabei hat ein Touchscreen auch im Vergleich zu einem mehrere Berührungssensoren, konkret einen resistiven und einen kapazitiven Berührungssensor, aufweisenden, ebenso zur Bediensicherheit beitragenden Touchscreen des Standes der Technik den Vorteil, dass "Force Touch", also die Ausgestaltung mit einem Berührungssensor und einem Kraftsensor, im Gegensatz zu dem zwei Berührungssensoren nutzenden Touchscreen die Bildqualität der Darstellung auf dem Touchscreen nicht beeinträchtigt. Ein weiterer Vorteil der Verwendung eines Touchscreens, der zusätzlich wenigstens einen Kraftsensor aufweist, ist, dass letztlich die Software für derartige Ansätze bereits existiert, mithin auch hier Aufwand eingespart werden kann. Touchscreens haben den weiteren Vorteil, dass sie auf besonders einfache Art und Weise auch die Vornahme weiterer, nicht auf derartige, Aufmerksamkeit für die Komponente und/oder den Patienten benötigender Bedienhandlungen angewendet werden kann, bei denen sich also der Bediener voll auf das auf dem Touchscreen Dargestellte konzentrieren kann, gegebenenfalls auch muss. Ein Beispiel hierfür ist die Einstellung von Aufnahmeparametern für einen anstehenden Bildaufnahmevorgang der medizinischen Bildgebungseinrichtung. Dann ist mithin das erfindungsgemäß vorgesehene Bedienmittel ausreichend, um alle notwendigen Bedienhandlungen an der medizinischen Bildgebungseinrichtung durchzuführen, ohne dass weitere Bedienmittel der Bedieneinrichtung zwangsläufig vorgesehen werden müssten. Dies hat eine vereinfachte, intuitive und kostengünstig realisierbare Bedieneinrichtung zur Folge.

Es sei angemerkt, dass derartige Anzeigeeinrichtungen, die der Bedienfläche zugeordnet sind, auch anderweitig realisiert werden können, beispielsweise als LED-Arrays und dergleichen.

Insbesondere bei Verwendung eines Touchscreens als Anzeigeeinrichtung sieht eine besonders vorteilhafte Weiterbildung der vorliegenden Erfindung vor, dass bei Vorsehen der dynamischen Definition von Bedienbereichen bei wenigstens einer ersten Bedienhandlung die Steuereinrichtung ferner zur Ansteuerung der Anzeigeeinrichtung zur Anzeige einer die Bedienbereiche beschreibenden Information ausgebildet ist. Das bedeutet, bei Durchführen der ersten Bedienhandlung können um die Berührposition herum, beispielsweise durch Anzeige entsprechender Bedienelemente, die Bedienbereiche auch optisch gekennzeichnet werden. Auf diese Weise kann der beispielsweise doch einen kurzen Blick auf das Bedienmittel werfende Bediener sich schnell und einfach versichern, den durch die erste Bedienhandlung aktivierten Betriebsmodus auch korrekt aktiviert zu haben. Zudem liefert eine entsprechende Anzeige der die Bedienbereiche beschreibenden Information eine Art Hilfsfunktion, insbesondere für neue Bediener. Auf diese Weise ist im Übrigen gleichzeitig eine flexible Gestaltung der Benutzeroberfläche gegeben, da letztlich keine Festlegung auf physikalische beziehungsweise physikalisch fest definierte Bedienelemente besteht, sondern abhängig von der Berührposition Bedienbereiche dynamisch passend zum durch die entsprechende erste Bedienhandlung angeforderten Betriebsmodus erzeugt beziehungsweise angepasst werden können.

Bei der medizinischen Bildgebungseinrichtung kann es sich, wie bereits dargelegt wurde, beispielsweise um eine Röntgeneinrichtung, insbesondere eine Computertomographieeinrichtung und/oder eine Magnetresonanzeinrichtung handeln. Konkret kann hierbei beispielsweise vorgesehen sein, dass bei einer als Computertomographieeinrichtung ausgebildeten medizinischen Bildgebungseinrichtung das Bedienmittel an einer Gantry und/oder bei einer als Magnetresonanzeinrichtung ausgebildeten medizinischen Bildgebungseinrichtung das Bedienmittel benachbart einer Patientenaufnahme an einer Hauptmagneteinheit angeordnet ist. An diesen Positionen wurden bereits normale, das bedeutet nicht kraftsensitive, Touchscreens vorgeschlagen, so dass sich auch die Position für das hier beschriebene Bedienmittel eignet. Selbstverständlich sind auch andere Positionen für das Bedienmittel denkbar, beispielsweise benachbart einer Patientenliege.

Eine der wenigstens einen Komponente ist vorzugsweise eine Patientenliege, da bei medizinischen Bildgebungseinrichtungen häufig eine Steuerung solcher beispielsweise über einen Motor als Aktor bewegbarer Patientenliegen erfolgt und zudem bei der Bewegung der Patientenliege mit darauf positionierten Patienten die Aufmerksamkeit diesem auch zuzuwenden ist. Weitere, insbesondere zusätzlich zu der Patientenliege vorgesehene Komponenten können eine Bildaufnahmekomponente und/oder eine Trägerkomponente und/oder eine Beleuchtungskomponente und/oder eine Klimatisierungskomponente, beispielsweise einen Patientenlüfter, umfassen. Beispielsweise bei Röntgeneinrichtungen mit einem C-Bogen existieren auch Trägerelemente, insbesondere der C-Bogen selbst, und/oder Bildaufnahmekomponenten, beispielsweise Röntgenstrahler und/oder Röntgendetektor, die über die Bedieneinrichtung bewegbar ausgestaltet sein können und, insbesondere bei einer denkbaren Kollisionsgefahr mit einem Patienten, bei der Steuerung über die Bedieneinrichtung im Auge zu behalten sind. Auch für derartige Komponenten eignet sich die erfindungsgemäße Ausgestaltung besonders.

Neben der medizinischen Bildgebungseinrichtung betrifft die vorliegende Erfindung auch ein Verfahren zum Betrieb einer medizinischen Bildgebungseinrichtung, insbesondere einer Computertomografieeinrichtung und/oder einer Magnetresonanzeinrichtung, wobei die Bildgebungseinrichtung wenigstens eine mittels eines Aktors bewegbare und/oder mittels eines Einstellmittels einstellbare Komponente, insbesondere umfassend wenigstens eine Patientenliege, eine Bedieneinrichtung zur Steuerung des Betriebs der wenigstens einen Komponente und eine der Bedieneinrichtung zugeordnete Steuereinrichtung aufweist. Bei einem solchen Verfahren ist erfindungsgemäß vorgesehen, dass als Bedienmittel der Bedieneinrichtung ein wenigstens einen Berührungssensor und wenigstens einen die Stärke der Berührung vermessenden Kraftsensor aufweisendes, berührungs- und kraftsensitives, flächiges Bedienmittel verwendet wird, wobei die Steuereinrichtung ausgebildet ist, bei Detektion wenigstens einer ersten Bedienhandlung abhängig von der Berührposition der ersten Bedienhandlung wenigstens zwei Bedienbereiche der Bedienfläche des Bedienmittels zu definieren, in denen jeweils unterschiedlichen Funktionalitäten zugeordnete zweite Bedienhandlungen detektierbar sind. Sämtliche Ausführungen bezüglich der erfindungsgemäßen Bildgebungseinrichtung lassen sich analog auf das erfindungsgemäße Verfahren übertragen, so dass auch mit diesem die bereits genannten Vorteile erhalten werden können. Insbesondere kann die Steuereinrichtung der medizinischen Bildgebungseinrichtung zur Durchführung des erfindungsgemäßen Verfahrens ausgebildet sein.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: eine Prinzipskizze einer erfindungsgemäßen medizinischen Bildgebungseinrichtung,
- Fig. 2: als schematischen Querschnitt den Aufbau eines Bedienmittels,
- Fig. 3: eine Skizze zur Illustration einer ersten Bedienhandlung,
- Fig. 4: eine Skizze zur Illustration einer zweiten Bedienhandlung, und
- Fig. 5: einen Ablaufplan eines Verfahrens zum Betrieb der Bildgebungseinrichtung.

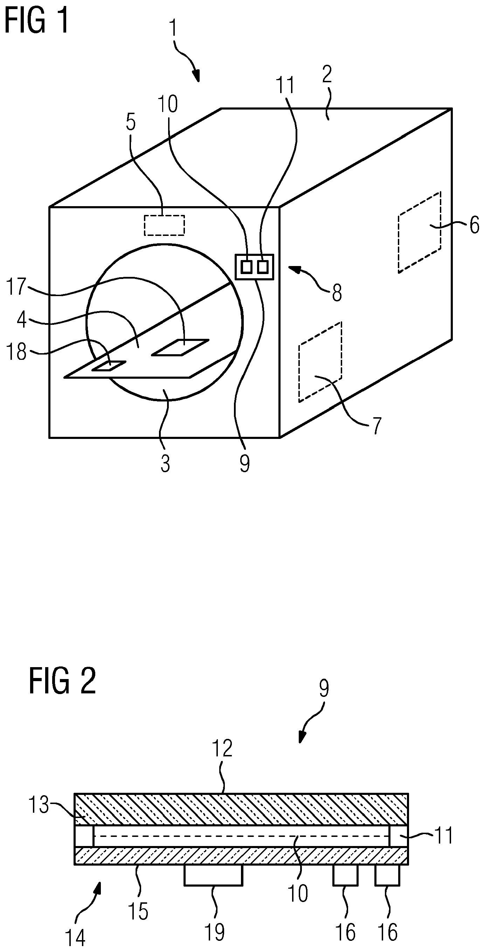

Fig. 1 zeigt eine vorliegend beispielhaft als Magnetresonanzeinrichtung ausgebildete medizinische Bildgebungseinrichtung 1 gemäß der vorliegenden Erfindung. Diese weist vorliegend eine Hauptmagneteinheit 2 auf, wie sie im Stand der Technik grundsätzlich bekannt ist und insbesondere den Grundfeldmagneten der Magnetresonanzeinrichtung aufnimmt. Die Hauptmagneteinheit 2 definiert eine zylindrische Patientenaufnahme 3, in die als bewegbare Komponente der Bildgebungseinrichtung 1 eine Patientenliege 4 eingefahren werden kann. Die medizinische Bildgebungseinrichtung 1 kann in diesem Zusammenhang ferner eine Beleuchtungskomponente 5 zur Beleuchtung des Inneren der Patientenaufnahme 3 und eine Klimatisierungskomponente 6, beispielsweise einen Patientenlüfter, aufweisen. Der Betrieb der medizinischen Bildgebungseinrichtung 1 wird mittels einer Steuereinrichtung 7 gesteuert. Um manuell Komponenten, beispielsweise die Patientenliege 4, die Beleuchtungskomponente 5 und/oder die Klimatisierungskomponente 6, bewegen und/oder einstellen zu können, weist die Bildgebungseinrichtung 1 ferner mindestens eine Bedieneinrichtung 8 auf, der die Steuereinrichtung 7 zugeordnet ist und die vorliegend ein als Touchscreen ausgebildetes, flächiges Bedienmittel 9 aufweist, welches nicht nur berührungssensitiv, sondern auch kraftsensitiv ist, so dass es sich um ein Force-Touch-Bedienmittel 9 handelt. Das Bedienmittel 9 weist mithin einen Berührungssensor 10 sowie wenigstens einen Kraftsensor 11 (Kraftaufnehmer) auf. Damit können nicht nur das Vorliegen und die Position von Berührungen der Bedienfläche des Bedienmittels 9 festgestellt werden, sondern auch deren Stärke.

Fig. 2 zeigt schematisch den Aufbau des Bedienmittels 9 genauer. Dieses weist eine die Bedienfläche 12 definierende Platte 13, insbesondere eine Glasplatte, auf, der, wie hier nur angedeutet und grundsätzlich bekannt, der beispielsweise kapazitiv messende Berührungssensor 10 zugeordnet ist. An den vier Ecken der Platte 13 ist jeweils ein Kraftsensor 11, vorliegend als Piezoelement ausgebildet, vorgesehen. Als Anzeigeeinrichtung 14 dient vorliegend das Display 15 des Touchscreens. Vorliegend weist das Bedienmittel 9 auch zwei Controller 16 auf, von denen je einer dem Berührungssensor 10 und den Kraftsensoren 11 zugeordnet sind. Dies stellt sicher, dass die Sensordaten der Sensoren 10, 11 zunächst unabhängig behandelt werden. Der den Kraftsensoren 11 zugeordnete Controller 16 beziehungsweise die Steuereinrichtung 7 sind ferner aufgrund der Krafteinträge in die einzelnen Kraftsensoren 11 ausgebildet, nicht nur die Stärke der Berührung zu ermitteln, sondern auch eine Berührposition schlusszufolgern, so dass die Berührposition mithin redundant sowohl durch den Berührungssensor 10 als auch durch die Kraftsensoren 11 ermittelt wird. Auf diese Weise kann zu einer funktional sicheren Auslegung der Bedieneinrichtung beigetragen werden, indem Bedienhandlungen nur bei innerhalb eines Toleranzbereichs übereinstimmenden Berührpositionen des Berührungssensors 10 und der Kraftsensoren 11 detektiert werden.

Wie im Folgenden noch näher dargelegt werden wird, ist der Betrieb des Bedienmittels 9 darauf ausgelegt, auch ohne Betrachtung des Bedienmittels 9 Bewegungen und/oder Einstellungen von Komponenten anzufordern. Soll beispielsweise die Patientenliege 4 mittels des hier nur angedeuteten Aktors 17 manuell gesteuert bewegt werden, ist davon auszugehen, dass der Bediener den Blick auf die Patientenliege 4 und den darauf positionierten Patienten gerichtet hält. Dies kann sich auch auf die Einstellung anderer Komponenten, beispielsweise der Beleuchtungskomponente 5 und/oder der Klimatisierungskomponente 6, beziehen.

Um dennoch zumindest bei nicht offensichtlich durch die Bewegung und/oder Einstellung der jeweiligen Komponente erkennbaren Steuermaßnahmen die erfolgreiche Detektion und/oder Ausführung einer einer Steuermaßnahme zugeordneten Bedienhandlung an dem Bedienmittel 9 anzeigen zu können, obwohl der Bediener den Blick nicht auf die Anzeigeeinrichtung 14 gerichtet hat, kann die medizinische Bildgebungseinrichtung 1 ferner weitere akustische und/oder haptische und/oder optische Anzeigemittel 18, 19 aufweisen, wobei es sich vorliegend bei dem Anzeigemittel 18 auch um ein optisches Anzeigemittel handeln kann, welches gezielt im Blickfeld des Bedieners auf den Patienten und die Patientenliege 4 angeordnet ist. Bei dem Anzeigemittel 19 kann es sich um ein akustisches und/oder haptisches Anzeigemittel 19 handeln. Bei einem haptischen Anzeigemittel 19 spürt der Bediener das haptische Feedback bei der Benutzung des Bedienmittels 9 unmittelbar - auch ein akustisches Feedback kann er ohne Blickabwendung von dem Patienten beziehungsweise der Komponente wahrnehmen.

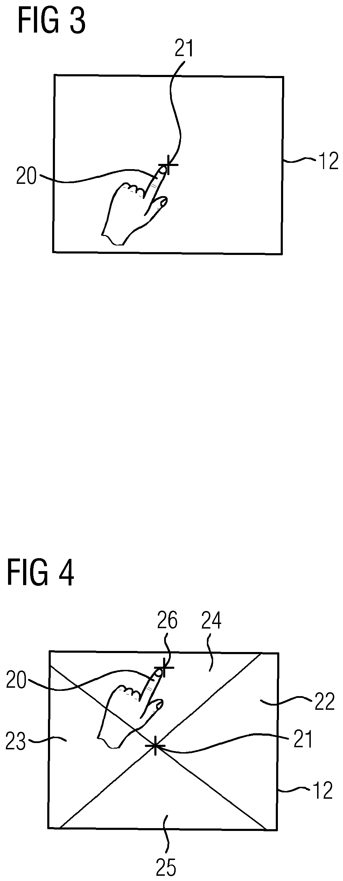

Die Figuren 3 und 4 erläutern eine mögliche Nutzung des Bedienmittels 9 mittels der Steuereinrichtung 7 genauer. Dabei befindet sich das Bedienmittel 9 vor den Geschehnissen der Fig. 3 beispielsweise in einem Ruhemodus, in dem auf der Anzeigeeinrichtung 15 keine Anzeige oder eine dieses verdeutlichende Anzeige vorgesehen sein kann. Mittels eines Fingers 20 führt der Bediener nun, den Blick weiterhin auf den Patienten und/oder die Komponente gerichtet, eine erste Bedienhandlung an einer beliebigen Berührposition 21 aus. Damit die erste Bedienhandlung als Bedienhandlung überhaupt akzeptiert wird, werden zudem einige Sicherheitsabfragen zur Herstellung der Erstfehlersicherheit durchgeführt, insbesondere, ob eine Mindestberührkraft und/oder eine Mindestberührdauer überschritten sind und ob sowohl der Berührungssensor 10 als auch die Kraftsensoren 11 innerhalb eines Toleranzbereichs dieselbe Berührposition anzeigen.

Dabei können verschiedene erste Bedienhandlungen, die möglichst einfach gehalten sind und sich gezielt auf eine beliebige Berührposition 21 beziehen, denkbar sein, um unterschiedliche, insbesondere auf unterschiedliche Komponenten bezogene Betriebsmodi zu aktivieren. Beispielsweise kann die erste Bedienhandlung einen "Doppelklick", also ein innerhalb eines Zeitraums erfolgendes, zweifaches Berühren und/oder ein mindestens für eine bestimmte Vorgabezeitdauer vorliegendes und/oder eine Vorgabeberührkraft überschreitendes, andauerndes Berühren der beliebigen Berührposition 21 umfassen.

Fig. 4 zeigt die Situation nach der ersten Bedienhandlung an der beliebigen Berührposition 21. Die Bedienfläche 12 ist dabei in diesem Beispiel in vier Bedienbereiche 22, 23, 24, 25 aufgeteilt worden, wobei sich jeweils die Bedienbereiche 22 und 23 sowie 24 und 25 bezüglich der Berührposition 21 gegenüberliegen. Die Bedienbereiche 22 bis 25 werden mittels der Anzeigeeinrichtung 14 anhand einer dynamisch erzeugten Information (hier der Übersichtlichkeit nicht näher dargestellt) dargestellt, wobei die Information nicht nur die Begrenzungen der Bedienbereiche 22 bis 25 enthält, sondern auch deren Zuordnung, um insbesondere neuen Bedienern durch einen kurzen Blick auf das Bedienmittel 9 zu ermöglichen, eine Bedienhilfe zu erhalten. Eine Orientierung ist aufgrund des Ausgehens von der Berührposition 21 der ersten Bedienhandlung ja bereits gegeben.

Soll beispielsweise eine Bewegung der Patientenliege 4 in dem nun aktivierten Betriebsmodus gesteuert werden, können die sich gegenüberliegenden Bedienbereiche 22 und 23 jeweils einem Hereinfahren und einem Herausfahren aus der Patientenaufnahme 3 entsprechen; die sich ebenfalls gegenüberliegenden oberen und unteren Bedienbereiche 24 und 25 können eine Steuerung der Höhe der Patientenliege 4 ermöglichen.

Nimmt nun der Bediener, wie anhand des wiederum dargestellten Fingers 20 und einer weiteren Berührposition 26 im Bedienbereich 24 angedeutet, eine zweite Bedienhandlung vor, ergibt sich deren Wirkung aus der Zuordnung zum Bedienbereich 22 bis 25. Die von den Kraftsensoren 11 erfasste Stärke der Berührung bei der zweiten Bedienhandlung bestimmt die Bewegungsgeschwindigkeit, mit der die Steuereinrichtung 7 bei Erkennen der zweiten Bedienhandlung die Patientenliege 4, beispielsweise unter Verwendung des Aktors 17, bewegt.

Wurde im Bild der Fig. 3 eine andere erste Bedienhandlung an der beliebigen Berührposition 21 durchgeführt, kann ein anderer Betriebsmodus aktiviert werden, beispielsweise ein Patientenkomfortmodus, bei dem die Bedienbereiche 22 bis 25 anderen Funktionalitäten zugeordnet sein können, beispielsweise die Bedienbereiche 22 und 23 der Einstellung der Stärke der Klimatisierungskomponente 6 und die Bedienbereiche 24 und 25 der Einstellung der Helligkeit der Beleuchtungskomponente 5.

Neben den hier beschriebenen Beispielen sind selbstverständlich auch eine Vielzahl anderer Bedienhandlungen, denen jeweils eine Steuermaßnahme der Steuereinrichtung 7 zugordnet ist, denkbar, insbesondere Gesten bzw. Force-Gesten, Multi-Touch-Bedienhandlungen und dergleichen. In jedem Fall ist die Steuereinrichtung 7 im hier dargestellten Ausführungsbeispiel auch ausgebildet, zur Erkennung zumindest mancher Bedienhandlungen den zeitlichen Verlauf der Berührkraft auszuwerten, um beispielsweise bei einem impulsartigen Berührkraftverlauf eine Notfall-Bedienhandlung, insbesondere einen Not-Bewegungsstopp, zu erkennen. Ein impulsartiger Berührkraftverlauf über die Zeit zeichnet sich dadurch aus, dass bei einer einen Steigungsschwellwert überschreitenden Steigung und/oder einen Zeitschwellwert unterschreitender Zeit eine einen Kraftschwellwert überschreitende Berührkraft erreicht wird. Die entsprechenden Schwellwerte sind dabei beispielsweise so zu wählen, dass ein Schlagen auf das Bedienmittel 9 als impulsartig erkannt wird.

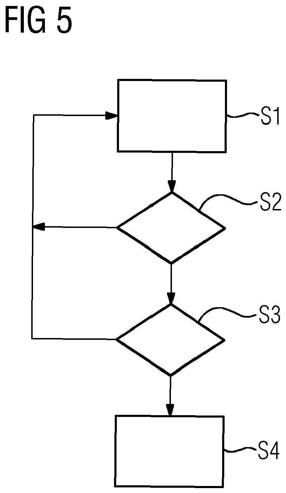

Fig. 5 zeigt schließlich einen Ablaufplan eines Verfahrens zum Betrieb des Bedienmittels 9 seitens der Steuereinrichtung 7. In einem Schritt S1 werden dabei Sensordaten des Berührungssensors 10 und der Kraftsensoren 11 aufgenommen, die im Fall einer vorliegenden Berührung eine Berührposition und eine Berührkraft, also die Stärke der Berührung, beschreiben. Im Fall von Gesten können solche Sensordaten auch zeitlich akkumuliert werden.

In einem Schritt S2 werden diverse Überprüfungen hinsichtlich der Bediensicherheit vorgenommen. So wird zum einen überprüft, ob die von den Sensoren 11 und dem Berührungssensor 10 ermittelte Berührposition innerhalb des Toleranzbereichs übereinstimmen. Eine weitere Bedingung stellt eine Mindestberührkraft dar, eine dritte Bedingung beispielsweise eine Mindestberührdauer. Sind diese Bedingungen nicht alle erfüllt, wird nicht zur Erkennung von Bedienhandlungen im Schritt S3 fortgeschritten. Das bedeutet, der Schritt S3 wird nur erreicht, wenn im Schritt S2 alle Bedingungen erfüllt sind, um so eine Erstfehlersicherheit zu erreichen.

Im Schritt S3 werden die ermittelten Berührpositionen und Berührkräfte, gegebenenfalls zeitlich akkumuliert, dann ausgewertet, um bestimmte Bedienhandlungen, denen Steuermaßnahmen zugeordnet sind, gegebenenfalls kontextbezogen, zu detektieren. Wird eine Bedienhandlung erkannt, wird mit dem Schritt S4 fortgefahren, in dem dann die entsprechende zugeordnete Steuermaßnahme ausgeführt wird und gegebenenfalls Anzeigemittel 18, 19 und/oder die Anzeigeeinrichtung 14 angesteuert werden, um das Erkennen der Bedienhandlung und die Durchführung der Steuermaßnahme anzuzeigen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Medizinische Bildgebungseinrichtung (1), insbesondere Computertomographieeinrichtung und/oder Magnetresonanzeinrichtung, aufweisend wenigstens eine mittels eines Aktors (17) bewegbare und/oder mittels eines Einstellmittels einstellbare Komponente, insbesondere umfassend wenigstens eine Patientenliege (4), weiterhin aufweisend eine Bedieneinrichtung (8) zur Steuerung des Betriebs der wenigstens einen Komponente und eine der Bedieneinrichtung (8) zugeordnete Steuereinrichtung (7), wobei die Bedieneinrichtung (8) ein wenigstens einen Berührungssensor (10) und wenigstens einen die Stärke der Berührung vermessenden Kraftsensor (11) aufweisendes, berührungs- und kraftsensitives, flächiges Bedienmittel (9) aufweist, **dadurch gekennzeichnet, dass** die Steuereinrichtung (7) ausgebildet ist, bei Detektion wenigstens einer ersten Bedienhandlung abhängig von der Berührposition (21) der ersten Bedienhandlung wenigstens zwei Bedienbereiche (22, 23, 24, 25) der Bedienfläche (12) des Bedienmittels (9) zu definieren, in denen jeweils unterschiedlichen Funktionalitäten zugeordnete zweite Bedienhandlungen detektierbar sind.

2. Medizinische Bildgebungseinrichtung (1) nach Anspruch 1, **dadurch gekennzeichnet, dass** dem Kraftsensor (11) und dem Berührungssensor (10) jeweils eigene Controller (16) zugeordnet sind und/oder die Steuereinrichtung (7) zum Detektieren wenigstens einer möglichen, auf die Bewegung einer Komponente bezogenen Bedienhandlung nur bei insbesondere innerhalb eines Toleranzbereichs übereinstimmenden Berührpositionsdaten des Berührungssensors (10) und des wenigstens einen auch zur Berührpositionsmessung ausgebildeten Kraftsensors (11) und/oder nur bei Überschreiten einer Mindestberührkraft und/oder einer Mindestberührzeitdauer ausgebildet ist.

3. Medizinische Bildgebungseinrichtung (1) nach Anspruch 2, **dadurch gekennzeichnet, dass** zur kraftsensorseitigen Berührpositionsmessung das Bedienmittel (9) mehrere Kraftsensoren (11) an unterschiedlichen Verbaupositionen aufweist, deren jeweilig gemessene Teilkräfte in dem zugeordneten Controller (16) und/oder der Steuereinrichtung (7) zur Ermittlung einer Berührposition (21, 26) und einer Berührkraft ausgewertet werden.

4. Medizinische Bildgebungseinrichtung (1) nach einem der vorangehenden Ansprüche, **dadurch gekennzeichnet, dass** die Steuereinrichtung (7) zur insbesondere stufenlosen Anpassung wenigstens eines einer Bedienhandlung zugeordneten Steuerparameters, insbesondere einer Bewegungsgeschwindigkeit, in Abhängigkeit von der zu der Bedienhandlung gemessenen Berührkraft ausgebildet ist und/oder die Steuereinrichtung (7) zur Unterscheidung von Bedienhandlungen aufgrund einer Berührkraft und/oder Berührdauer zur Erkennung eines innerhalb eines Zeitraums erfolgenden, zweifachen Berührens als Bedienhandlung, insbesondere zum Wechsel eines Bedienmodus, ausgebildet ist.

5. Medizinische Bildgebungseinrichtung (1) nach einem der vorangehenden Ansprüche, **dadurch gekennzeichnet, dass** die Steuereinrichtung (7) zur Auswertung eines Berührkraftverlaufs über die Zeit zur Detektion einer Bedienhandlung ausgebildet ist.

6. Medizinische Bildgebungseinrichtung (1) nach Anspruch 5, **dadurch gekennzeichnet, dass** die Steuereinrichtung (7) zur Detektion einer Notfall-Bedienhandlung, insbesondere eines Not-Bewegungsstopps, bei einem impulsartigen Berührkraftverlauf, bei dem mit einer einen Steigungsschwellwert überschreitenden Steigung und/oder in einer einen Zeitschwellwert unterschreitenden Zeit eine einen Kraftschwellwert überschreitende Berührkraft erreicht wird, ausgebildet ist.

7. Medizinische Bildgebungseinrichtung (1) nach einem der vorangehenden Ansprüche, **dadurch gekennzeichnet, dass** durch die Steuereinrichtung (7) wenigstens zwei bezüglich der Berührposition (21) der ersten Bedienhandlung gegenüberliegende Bedienbereiche (22, 23, 24, 25) der Ansteuerung unterschiedlicher Bewegungsrichtungen und/oder Einstellrichtungen einer der wenigstens einen Komponente, insbesondere der Patientenliege (4), zugeordnet sind, wobei insbesondere die Berührkraft einer zur Bewegung der Komponente vorgesehenen zweiten Bedienhandlung steuereinrichtungsseitig zur Ermittlung einer Bewegungsgeschwindigkeit und/oder Einstellungsgeschwindigkeit auswertbar ist.

8. Medizinische Bildgebungseinrichtung (1) nach einem der vorangehenden Ansprüche, **dadurch gekennzeichnet, dass** die Steuereinrichtung (7) zur Anwahl eines Bedienbereiches durch Auswertung der Richtung eines Ziehens ausgehend von der Berührposition (21) nach der ersten Bedienhandlung ausgebildet ist.

9. Medizinische Bildgebungseinrichtung (1) nach einem der vorangehenden Ansprüche, **dadurch gekennzeichnet, dass** die Bedieneinrichtung (8) ferner wenigstens ein dem Bedienmittel (9) zugeordnetes, akustisches und/oder haptisches und/oder optisches Anzeigemittel (18, 19) zur Anzeige und/oder Bestätigung wenigstens einer Bedienhandlung aufweist.

10. Medizinische Bildgebungseinrichtung (1) nach Anspruch 9, **dadurch gekennzeichnet, dass** das optische Anzeigemittel (18, 19) wenigstens einer Komponente zugeordnet und im Blickbereich einer das Bedienmittel (9) nutzenden Person zu der Komponente, entfernt von dem Bedienmittel (9) angeordnet ist.

11. Medizinische Bildgebungseinrichtung (1) nach einem der vorangehenden Ansprüche, **dadurch gekennzeichnet, dass** das Bedienmittel (9) eine die Bedienfläche (12) wenigstens teilweise abdeckende Anzeigeeinrichtung (14) aufweist, insbesondere dass das Bedienmittel (9) als ein Touchscreen ausgebildet ist.

12. Medizinische Bildgebungseinrichtung (1) nach Anspruch 11, **dadurch gekennzeichnet, dass** bei einer Ausgestaltung nach Anspruch 7 die Steuereinrichtung (7) ferner zur Ansteuerung der Anzeigeeinrichtung (14) zur Anzeige einer die Bedienbereiche (22, 23, 24, 25) beschreibenden Information ausgebildet ist.

13. Medizinische Bildgebungseinrichtung (1) nach einem der vorangehenden Ansprüche, **dadurch gekennzeichnet, dass** bei einer als Computertomographieeinrichtung ausgebildeten medizinischen Bildgebungseinrichtung (1) das Bedienmittel (9) an einer Gantry und/oder bei einer als Magnetresonanzeinrichtung ausgebildeten medizinischen Bildgebungseinrichtung (1) das Bedienmittel (9) benachbart einer Patientenaufnahme (3) an einer Hauptmagneteinheit (2) angeordnet ist und/oder die Komponenten, insbesondere zusätzlich zu der Patientenliege (4), eine Bildaufnahmekomponente und/oder eine Trägerkomponente und/oder eine Beleuchtungskomponente (5) und/oder eine Klimatisierungskomponente (6) umfassen.

14. Verfahren zum Betrieb einer medizinischen Bildgebungseinrichtung (1), insbesondere Computertomographieeinrichtung und/oder Magnetresonanzeinrichtung, wobei die Bildgebungseinrichtung (1) wenigstens eine mittels eines Aktors (17) bewegbare und/oder mittels eines Einstellmittels einstellbare Komponente, insbesondere umfassend wenigstens eine Patientenliege (4), und zudem eine Bedieneinrichtung (8) zur Steuerung des Betriebs der wenigstens einen Komponente aufweist, wobei als Bedienmittel (9) der Bedieneinrichtung (8) ein wenigstens einen Berührungssensor (10) und wenigstens einen die Stärke der Berührung vermessenden Kraftsensor (11) aufweisendes, berührungs- und kraftsensitives, flächiges Bedienmittel (9) verwendet wird, **dadurch gekennzeichnet, dass** die Steuereinrichtung (7) ausgebildet ist, bei Detektion wenigstens einer ersten Bedienhandlung abhängig von der Berührposition (21) der ersten Bedienhandlung wenigstens zwei Bedienbereiche (22, 23, 24, 25) der Bedienfläche (12) des Bedienmittels (9) zu definieren, in denen jeweils unterschiedlichen Funktionalitäten zugeordnete zweite Bedienhandlungen detektierbar sind.

## Claims

1. Medical imaging device (1), in particular a computed tomography device and/or magnetic resonance device, having at least one component able to be moved by means of an actuator (17) and/or adjusted by means of an adjustment means, in particular comprising at least one patient couch (4), furthermore having an operating device (8) for controlling the operation of the at least one component and a control device (7) assigned to the operating device (8), wherein the operating device (8) has a touch-sensitive and force-sensitive means of operation (9) having at least one touch sensor (10) and at least one force sensor (11) measuring the strength of the touch, **characterised in that** the control device (7) is embodied, on detection of at least one first operating action, depending on the touch position (21) of the first operating action, to define at least two operating areas (22, 23, 24, 25) of the operating surface (12) of the means of operation (9), in which second operating actions assigned to different functionalities are able to be detected in each case.

2. Medical imaging device (1) according to claim 1, **characterised in that** separate controllers (16) are assigned to the force sensor (11) and the touch sensor (10) in each case and/or that the control device (7) is embodied for detecting at least one possible operating action related to the movement of a component only for touch position data in particular within a tolerance range of matching touch position data of the touch sensor (10) and of the at least one force sensor (11) also embodied for touch position measurement and/or only when a minimum touch force and/or a minimum touch duration is exceeded.

3. Medical imaging device (1) according to claim 2, **characterised in that**, for force-sensor-side touch position measurement, the means of operation (9) has a number of force sensors (11) installed at different positions, of which the measured part forces in each case are evaluated in the assigned controller (16) and/or the control device (7) to establish a touch position (21, 26) and a touch force.

4. Medical imaging device (1) according to one of the preceding claims, **characterised in that** the control device (7) is embodied for in particular stepless adaptation of a control parameter assigned to at least one operating action, in particular a speed of movement, as a function of the touch force measured for the operating action and/or that the control device (7) is embodied to distinguish operating actions on the basis of a touch force and/or touch duration for recognising a double touch taking place with a period of time as an operating action, in particular for switching a mode of operation.

5. Medical imaging device (1) according to one of the preceding claims, **characterised in that** the control device (7) is embodied for evaluating the course of a touch force over time for detection of an operating action.

6. Medical imaging device (1) according to claim 5, **characterised in that** the control device (7) is embodied for detection of an emergency operating action, in particular an emergency movement stop, with a pulse type course of a touch force, in which with an increase exceeding an increase threshold value and/or in a time falling below a time threshold value a touch force exceeding a force threshold value is reached.

7. Medical imaging device (1) according to one of the preceding claims, **characterised in that** at least two opposite operating areas (22, 23, 24, 25) relating to the touch position (21) of the first operating action are assigned to the activation of different directions of movement and/or directions of adjustment of one of the at least one components, in particular the patient couch (4), by the control device (7), wherein in particular the touch force of a second operating action provided for moving the component is able to be evaluated on the control device side to establish a speed of movement and/or speed of adjustment.

8. Medical imaging device (1) according to one of the preceding claims, **characterised in that** the control device (7) is embodied for selecting an operating area by evaluating the direction of a drag starting from the touch position (21) after the first operating action.

9. Medical imaging device (1) according to one of the preceding claims, **characterised in that** the operating device (8) further has at least one acoustic and/or haptic and/or optical display means (18, 19) assigned to the means of operation (9) for displaying and/or acknowledging at least one operating action.

10. Medical imaging device (1) according to claim 9, **characterised in that** the optical display means (18, 19) is assigned to at least one component and is arranged remote from the means of operation (9) in the field of vision of a person using the means of operation (9) for the component.

11. Medical imaging device (1) according to one of the preceding claims, **characterised in that** the means of operation (9) has a display device (14) at least partly covering the operating surface (12), in particular that the means of operation (9) is embodied as a touchscreen.

12. Medical imaging device (1) according to claim 11, **characterised in that**, with an embodiment according to claim 7 the control device (7) is further embodied for activation of the display device (14) for displaying information describing the operating areas (22, 23, 24, 25).

13. Medical imaging device (1) according to one of the preceding claims, **characterised in that**, with a medical imaging device (1) embodied as a computed tomography device, the means of operation (9) is arranged on a gantry and/or with a medical imaging device (1) embodied as a magnetic resonance device, the means of operation (9) is arranged adjacent to a patient receiving area (3) on a main magnet unit (2) and/or that the components, in particular in addition to the patient couch (4), comprise an imaging component and/or a carrier component and/or a lighting component (5) and/or an air conditioning component (6).

14. Method for operating a medical imaging device (1), in particular a computed tomography device and/or magnetic resonance device, wherein the imaging device (1) has at least one component able to be moved by means of an actuator (17) and/or able to be adjusted by means of an adjustment means, in particular comprising at least one patient couch (4), and also an operating device (8) for controlling the operation of the at least one component, wherein a touch-sensitive and force-sensitive, flat means of operation (9) having at least one touch sensor (10) and at least one force sensor (11) measuring the strength of the touch is used as the means of operation (9) of the operating device (8), **characterised in that** the control device (7) is embodied, on detection of at least one first operating action, depending on the touch position (21) of the first operating action, to define at least two operating areas (22, 23, 24, 25) of the operating surface (12) of the means of operation (9), in which second operating actions assigned to different functionalities are able to be detected in each case.

## Revendications

1. Dispositif (1) d'imagerie médicale, notamment dispositif de tomodensitométrie assistée par ordinateur et/ou dispositif à résonnance magnétique, comportant au moins un élément mobile à l'aide d'un actionneur (17) et/ou réglable à l'aide d'au moins un moyen de réglage, comprenant notamment au moins une couchette (4) de patient comportant en outre un dispositif (8) de service pour commander le fonctionnement du au moins un élément et un dispositif (7) de commande associé au dispositif (8) de service, le dispositif (8) de service ayant un moyen (9) de service à deux dimensions, sensible au contact et à une force et ayant au moins un capteur (10) de contact et au moins un capteur (11) de force mesurant la force du contact, **caractérisé en ce que** le dispositif (7) de commande est constitué pour, à la détection d'au moins une première manipulation de service, définir, en fonction de la position (21) de contact de la première manipulation de service, au moins deux parties (22, 23, 24, 25) de service de la surface (12) de service du moyen (9) de service, dans lesquelles des deuxièmes manipulations de service associées chacune à des fonctionnalités différentes peuvent être détectées.

2. Dispositif (1) d'imagerie médicale suivant la revendication 1, **caractérisé en ce qu'**au capteur (11) de force et au capteur (10) de contact sont associées respectivement leurs propres unités (16) de commande et/ou le dispositif (7) de commande est constitué pour la détection d'au moins une manipulation de service possible, rapportée au déplacement d'un élément, seulement pour des données de position de contact coïncidant, notamment dans une plage de tolérance, du capteur (10) de contact et du au moins un capteur (11) de force constitué également pour la mesure de la position de contact et/ou seulement si une force de contact minimum et/ou une durée de contact minimum est dépassée.

3. Dispositif (1) d'imagerie médicale suivant la revendication 2, **caractérisé en ce que**, pour la mesure de la position de contact de la part du capteur de force, le moyen (9) de service a plusieurs capteurs (11) de force en des positions de pose différentes, dont les forces partielles respectives mesurées sont, pour la détermination d'une position (21, 26) de contact et d'une force de contact, exploitées dans l'unité (16) de commande associée et/ou le dispositif (7) de commande.

4. Dispositif (1) d'imagerie médicale suivant l'une des revendications précédentes, **caractérisé en ce que** le dispositif (7) de commande est constitué pour l'adaptation, notamment sans palier, d'au moins un paramètre de commande, associé à un traitement de service, notamment d'une vitesse de déplacement, en fonction de la force de contact mesurée à la manipulation de service et/ou le dispositif (7) de commande est constitué pour différencier des manipulations de service sur la base d'une force de contact et/ou d'une durée de contact, afin de reconnaître un contact (12) s'effectuant dans un laps de temps comme manipulation de service, notamment pour le remplacement d'un mode de service.

5. Dispositif (1) d'imagerie médicale suivant l'une des revendications précédentes, **caractérisé en ce que** le dispositif (7) de commande est constitué pour l'exploitation d'une courbe de force de contact en fonction du temps, afin de détecter une manipulation de service.

6. Dispositif (1) d'imagerie médicale suivant la revendication 5, **caractérisé en ce que** le dispositif (7) de commande est constitué pour la détection d'une manipulation de service d'urgence, notamment d'un arrêt de déplacement d'urgence, dans une courbe de force de contact de type à impulsion, dans laquelle on atteint, par une pente dépassant une valeur de seuil de pente et/ou dans un temps dépassant une valeur de seuil de temps, une force de contact dépassant une valeur de seuil de force.

7. Dispositif (1) d'imagerie médicale suivant l'une des revendications précédentes, **caractérisé en ce que** par le dispositif (7) de commande, au moins deux parties (22, 23, 24, 25) de service, opposées par rapport à la position (21) de contact de la première manipulation de service, sont associées à la commande de directions de déplacement et/ou de directions de réglage différents de l'un des au moins un élément, notamment de la couchette (4) du patient dans lequel notamment la force de contact d'une deuxième manipulation de service prévue pour le déplacement de l'élément peut être évaluée de la part du dispositif de commande pour la détermination d'une vitesse de déplacement et/ou d'une vitesse de réglage.

8. Dispositif (1) d'imagerie médicale suivant l'une des revendications précédentes, **caractérisé en ce que** le dispositif (7) de commande est constitué pour le choix d'une partie de service en exploitant la direction d'une traction à partir de la position (21) de contact après la première manipulation de service.

9. Dispositif (1) d'imagerie médicale suivant l'une des revendications précédentes, **caractérisé en ce que** le dispositif (8) de service a en outre au moins un moyen (18, 19) d'affichage acoustique et/ou optique associé au moyen (9) de service pour l'affichage et/ou la confirmation d'au moins une manipulation de service.

10. Dispositif (1) d'imagerie médicale suivant la revendication 9, **caractérisé en ce que** le moyen (18, 19) d'affichage optique est associé à au moins un élément et est disposé en étant éloigné du moyen (9) de service par rapport à l'élément dans la direction de vision d'une personne utilisant le moyen (9) de service.

11. Dispositif (1) d'imagerie médicale suivant l'une des revendications précédentes, **caractérisé en ce que** le moyen (9) de service a un dispositif (14) d'affichage recouvrant la surface (12) de service au moins en partie, le moyen (9) de service étant constitué notamment sous la forme d'un écran tactile.

12. Dispositif (1) d'imagerie médicale suivant la revendication 11, **caractérisé en ce que** dans un mode de réalisation suivant la revendication 7, le dispositif (7) de commande est constitué en outre pour commander le dispositif (14) d'affichage pour l'affichage d'une information décrivant les parties (22, 23, 24, 25) de service.

13. Dispositif (1) d'imagerie médicale suivant l'une des revendications précédentes, **caractérisé en ce que** dans un dispositif (1) d'imagerie médicale constitué sous la forme d'un dispositif de tomodensitométrie assistée par ordinateur, le moyen (9) de service est monté sur un portique et/ou dans un dispositif (1) d'imagerie médicale constitué sous la forme d'un dispositif à résonnance magnétique, le moyen (9) de service est monté au voisinage d'un logement (3) du patient sur une unité (2) magnétique principale et/ou les éléments, notamment supplémentairement à la couchette (4) du patient, comprennent un élément de prise d'image et/ou un élément de support et/ou un élément (5) d'éclairage et/ou un élément (6) de conditionnement d'air.

14. Procédé pour faire fonctionner un dispositif (1) d'imagerie médicale, notamment un dispositif de tomodensitométrie assistée par ordinateur et/ou un dispositif à résonnance magnétique, le dispositif (1) d'imagerie comportant au moins un élément mobile à l'aide d'un actionneur (17) et/ou réglable à l'aide d'au moins un moyen de réglage, comprenant notamment au moins une couchette (4) de patient, comportant en outre un dispositif (8) de service pour commander le fonctionnement du au moins un élément et un dispositif (7) de commande associé au dispositif (8) de service, dans lequel on utilise, comme moyen (9) de service du dispositif (8) de service, un moyen (9) de service à deux dimensions, sensible au contact et à une force et ayant au moins un capteur (10) de contact et au moins un capteur (11) de force mesurant la force du contact, **caractérisé en ce que** le dispositif (7) de commande est constitué pour, à la détection d' au moins une première manipulation de service, définir, en fonction de la position (21) de contact de la première manipulation de service, au moins deux parties (22, 23, 24, 25) de service de la surface (12) de service du moyen (9) de service, dans lesquelles des deuxièmes manipulations de service associées chacune à des fonctionnalités différentes peuvent être détectées.
